# EUROPEAN PATENT SPECIFICATION

(11) **EP 2 218 411 B1**
(45) Date of publication and mention of the grant of the patent: **26.10.2011**
(21) Application number: 09180367.6
(22) Date of filing: 22.12.2009
(51) Int. Cl.: A61B 17/16, A61B 17/32

(54) **Retrograde cutter with rotating blade**
Rückläufige Schneidevorrichtung mit Drehklinge
Dispositif de découpe rétrograde avec lame rotative

(30) Priority: 29.12.2008 US 141183 P
(43) Date of publication of application: 18.08.2010
(73) Proprietor: Arthrex, Inc., Naples, FL 34108-1945 (US)
(72) Inventor: Sherman, Gary Scott, Naples, FL 34109 (US)
(74) Representative: Rosenquist, Per Olof

(56) References cited:
- EP-A2- 1 690 499
- US-A- 5 649 947
- US-A1- 2003 135 218
- US-A1- 2004 106 940
- US-A1- 2007 276 391

## Description

### FIELD OF THE INTENTION

The present invention relates to a mechanism for converting linear movement into rotational movement for a surgical instrument, such as a flip retrograde cutting instrument.

### BACKGROUND OF THE INVENTION

During arthroscopic surgery, a small incision is made in the skin covering the arthroscopic site or joint, and a cannula is inserted in the incision to provide a pathway for surgical instruments to be placed in the joint and manipulated through arthroscopic visualization. Surgical instruments inserted through cannulas must be long and thin - this presents limitations on instruments for cutting tissue, as the diameter of the cannula ordinarily limits the width of the cutting implement.

Retrograde drilling of sockets and tunnels for ACL reconstruction using a flip cutter is known and described, for example, in U.S. Patent Application Publ. No. 2009/0275950 A1 and EP 1987786. This prior published application describes a flip retrograde cutter having a blade, preferably a flip blade, that is configured to articulate between at least a first "straight" position, for example, substantially parallel to a longitudinal axis of the flip retrograde cutter, and at least a second "flip" position, for example, anon-parallel position relative to the longitudinal axis of the flip retrograde cutter. Using such a flip retrograde cutter, a recipient site socket can be created from the inside out, i.e., using a retrograde technique, with minimal incisions of distal cortices and reduced intraarticular bone fragmentation of tunnel rims.

The flip retrograde cutter described above may be employed in a retrograde manner to form a recipient socket (to accommodate an osteochondral transplant, or to allow retrograde fixation of a graft within two sockets, for example). Formation of the recipient socket begins by inserting the flip retrograde cutter in the "straight" configuration into the joint space, preferably from the outside in, through a small diameter tunnel. A locking tube of the instrument is then retracted so that the blade can be articulated into the "flip" configuration, i.e., into a position other than the "straight" position and preferably at about 90 degrees to the longitudinal axis of the instrument. The device is locked in the "flip" position by tightening the locking tube. A socket is created by conducing a drilling operation; i.e., by rotating the instrument, white the device is pulled outwardly.

U.S. 2007/0276391 discloses a bone resection device with two blades attached to a blade housing. The blades are not configured to extend between two positions and are also not configured for retrograde drilling of a bone tunnel or socket

U.S. 2004/0106940 relates to a device for accessing the intervertebral space to restore a three-dimensional configuration of the space, with or without fusing two adjacent vertebrae. A blade is attached to the distal end of a hinge by dowel and to an inner tube, and is configured to extend between two positions.. There is no disclosure or suggestion in this document of retrograde drilling of a bone tunnel or socket.

U.S. 5,649,947 teaches a surgical instrument having an elongate support shaft, a tip assembly permanently attached to the shaft, a cutting jaw which opens in a backward facing direction and has a fully open position in which the movable element extends outwardly from the longitudinal axis and toward a proximal end of the elongate shaft at an angle of approximately 60°

U.S. 2003/0135218 teaches a diskectomy instrument with a probe assembly having four blades designed to fit a concave portion of a particular vertebra. The blades are not configured, however, to extend between two positions, and they are also not configured to cut a bone tunnel or socket by drilling in a retrograde manner.

EP 1690499 teaches an apparatus for attaching sutures comprising an anchor body with an intruding edge that penetrates tissue when anchor is inserted for attachment to tissue. Upon insertion, the anchor may rotate in the hole so that the intruding edge penetrates the bony tissue forming the wall of the hole. This document does not disclose or suggest a rotating blade of a retrograde cutter, much less a rotating blade with all specific characteristics recited in claim 1.

A need exists for a mechanism that facilitates flipping of the blade in the above-described instrument by converting linear movement of the shaft into rotational movement of the cutter blade (without the need to manually flip the cutter blade into position within the joint).

### SUMMARY OF THE INVENTION

The present invention is defined in claim 1 and provides a cutting instrument that is designed to automatically convert libear movement of the shaft of the instrument into a rotational movement of the cutter tip (for example, a blade) of the instrument. The flip cutter instrument is provided with a mechanism (for example, a pin and a slot) that converts linear motion into rotational motion and locks the blade into position. The cutting blade is configured to engage the shaft of the instrument and to lock into the shaft. The cutting blade is articulated between at least a first "straight".position. (for example, about parallel to the longitudinal axis of the instrument) when the instrument cuts in an antegrade manner, and at least a second "flip" position (for example, a non-parallel position relative to the longitudinal axis of the instrument) when the instrument cuts in a retrograde manner. The cutting Made may be provided with a suture passing notch. The cutter instrument may also: include a button mechanism for controlling the linear movement.

The cutter of the present invention may be employed in an antegrade manner, or in a retrograde manner, or both in an antegrade and retrograde manner, to form a recipient socket (to accommodate an osteochondral transplant, or to allow retrograde fixation of a graft within two sockets, for example).

These and other features and advantages of the invention will be more apparent from the following detailed description that is provided in convection with the accompanying drawings and illustrated exemplary embodiments of the invention.

### BRIEF DESCRIPTION OF THE DRAWINGS

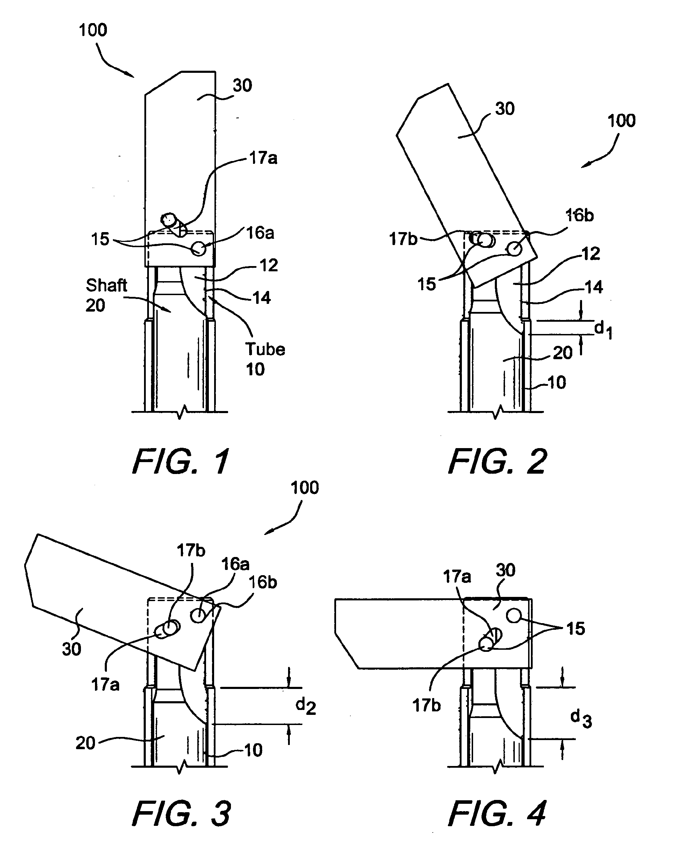

FIG. 1 illustrates a schematic cross-sectional view of a flip cutter according to a first embodiment of the present invention, and with the blade in the "straight" configuration.

. F1GS. 2 and 3 illustrate the flip cutter of FIG. 1, with the blade in sequential "flip" configurations.

FIG. 4 illustrates the flip cutter of FIG. 1, with the blade in the locked "flip" configuration.

FIG. 5 illustrates a perspective view of flip cutter according to a second embodiment of the present invention, and with the blade in the "flip" configuration.

FIG. 6 illustrates a side view of the flip cutter of FIG. 5, with the blade in the locked "flip" configuration.

FIG. 7 illustrates a top view of the nip cutter of FIG. 5, with the"blade in the locked "flip" configuration.

FIGS. 8 and 9 illustrate side views of the push button mechanism of the flip cutter of FIG. 5.

FIG. 10 schematically illustrates the formation ofa socket with the flip cutter of the present invention.

### DETAILED DESCRIPTION OF THIS INTENTION

The following description is provided to enable any person skilled in the art to make and use the invention and sets forth the best modes contemplated by the inventors of carrying out their invention. Various modifications, however, will remain readily apparent to those skilled in the art.

The present invention provides a flip cutter instrument that is designed to automatically convert linear movement of the shaft of the instrument into a rotational movement of the cutter tip (blade) of the instrument. The flip cutte instrument is provided with a mechanism (such as a pin and a slot, for example) that converts linear motion into rotational motion and locks the blade into position. The cutting Made is configured to engage the shaft of the instrument and to lock into the shaft. The blade is articulated between at least a first "straight" position (for-example, about parallel to the longitudinal axis of the instrument) and at least a second "flip" position (for example, a non-parallel position relative to the longitudinal axis of the instrument). The cutting blade may be provided with a suture passing notch.

The instrument may function in an antegrade or retrograde manner (when in the "flip" mode), or both in an antegrade or retrograde manner, to form a recipient socket (to accommodate an osteochondral transplant, or to allow retrograde fixation of a graft within two sockets, for example).

As described in more detail below, formation of the recipient socket begins by inserting an outer tube and an inner tube (i.e., a shaft) of the instrument into the joint space, preferably from the outside in, through a small diameter tunnel. A cutting blade is attached to both the outer tube and the inner tube of the instrument. The cutting blade may be provided with a suture passing notch in the blade. Once the shaft undergoes linear movement or linear motion, a mechanism (a pin and slot mechanism, for example) converts the linear movement of the shaft <in relation to the tube) into rotational movement of the cutting blade. The pin and slot mechanism also locks the blade on the outer tube when the blade is in the "flip" position. A socket is created by conducting a drilling operation while the device is pulled in a retrograde manner, as described, for example, in U.S. Patent Application Publ. No. 2008/0306483. Other methods of retrograde drilling known in the art may also be used to create the socket.

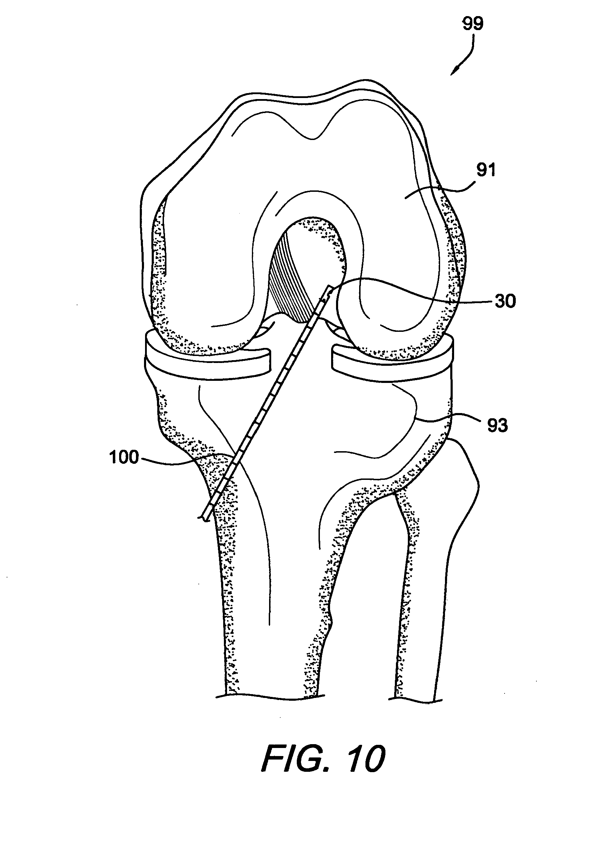

Referring now to the drawings, where like elements are designated by like reference numerals, Figures 1-9 illustrate various views of flip cutter 100, 200 of the present invention and at various stages of converting linear motion to rotational motion. Figure 10 illustrates a schematic view of the retrograde flip cutter 100 of Figures 1-4 provided in the vicinity of a knee, where ACL reconstruction is conducted according to the present invention. The flip cutter 100; 200 creates a recipient site socket from the inside out, i.e., using a retrograde technique, with minimal incisions of distal cortices and reduced intraarticular bone fragmentation of tunnel rims.

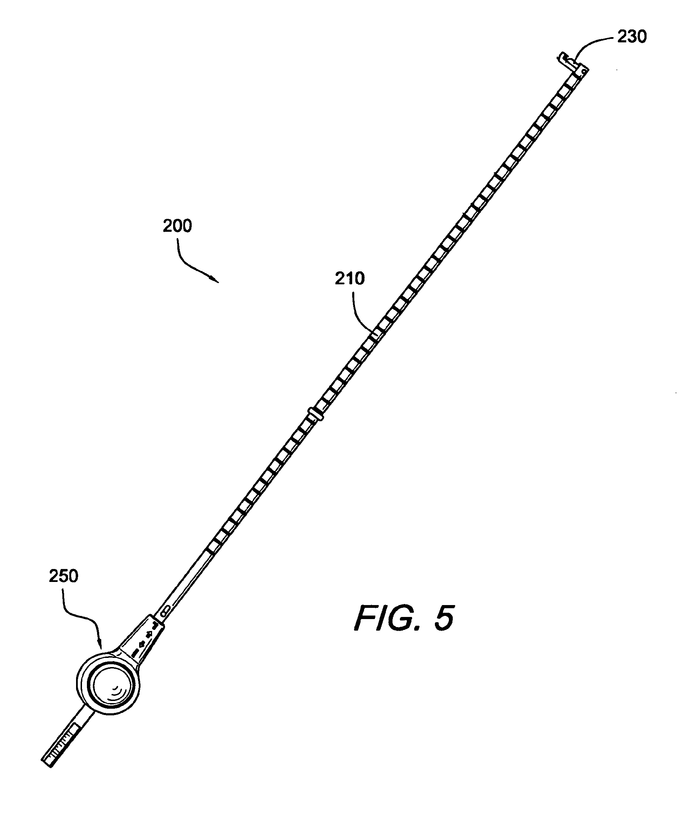

Figures 1-4 illustrate a first embodiment of flip cutter 100 of the present invention while Figures 5-9 illustrate a second embodiment of Hip cutter 200 of the present invention (provided with a push button mechanism for deployment, and' also with a suture passing notch in the cutting blade). As illustrated in Figures 1-4, flip cutter 100 includes a cannulated elongated outer tube 10 having a distal end 12 and a proximal end (not shown. Distal end 12 is provided (at its most distal part) with a mechanism 15 that is configured to engage a corresponding structure of blade 30 to be attached and securely engaged to the outer tube, 10.

The outer tube 10 of the retrograde cutter 100 hoses an inner tube or shaft, 20 with a diameter smaller than that of the outer tube 10. Blade 30 is provided at distal end 12 of the outer tube 10 and is connected to both the outer tube 10 and the inner shaft 20 by mechanism 15. In an exemplary embodiment, and as shown in Figure 1, blade 30 is pinned to the outer tube 10 and is also pinned to the shaft 20. Outer tube 10 is provided with a cutout 14 that allows movement of blade 15 within the cutout and relative to the outer tube 10. Blade 30 of the cutter 100 may have a body provided in various shapes and geometries, and may include cutting edges or surfaces also of various configurations.

In an exemplary embodiment, mechanism 15 comprises a pin and a slot that allow conversion of the linear movement of the shaft of the instrument into rotational movement of the cutter tip (cutting blade) of the instrument. For example, as shown in Figures 1-4, mechanism 15 may include a first pin hole 16a (or first pin slot 16a) with a first pin 16b connecting blade 30 to tube 10, where the first pin hole 16a permits only rotational movement, and a second pin hole 17a (or second pin slot 17a) with a second pin 17b connecting blade 30 to shaft 20, where the second pin hole 17a is a slot permitting rotational and sliding movement of blade 30 relative to the second pin 17b. As shown in Figures 2 and 3, when tube 10 is advanced in a linear direction parallel to the longitudinal axis of the flip cutter 100, the first pin pushes one side of the proximal end of blade 30 in the linear direction, white the second pin is permitted to slide in the slot of the second pin hole, thus permitting rotation of blade 30:

In use, blade 30 is attached to both the outer tube 10 and the inner tube 20 by engaging mechanism 15. The outer tube 10 and the inner tube 20, with the blade attached and locked in the "straight" configuration, are inserted into a joint from the distal side, until they are visible in the joint.

Once the instrument reaches the joint, a linear motion may be carried out so that one of the tubes 10, 20 advances relative to the other of the tubes 10, 20 (for example, the outer tube 10 advances relative to the inner shaft 20) by sequential distance d₁ (Figure 2), d₂ (Figure 3) and d₃ (Figure 4). At the point where outer tube 10 travels distance d₃ (Figure 4) relative to the inner tube 20 (or when the inner tube 20 travels distance d₃ relative to the outer tube 10), the blade 30 is in a locked and "flip" position, i.e., about perpendicular to the longitudinal axis of the instrument, In this manner, movement of the outer tube relative to the inner shaft (i.e., while traveling a distance between about 0 to about d₃) converts the linear motion of the tube into a rotational motion of blade 30.

Although Figures 1-4 illustrate blade 30 being articulated to the second position upon movement of tube 10 in a distal direction, it should be understood that other embodiments could include blade 30 being articulated to the second position upon movement of either shaft 20 or tube 10 in a proximal direction. In addition, although Figures 1-4 illustrate outer tube 10 being moved linearly, it should be understood that the articulation of blade 30 to the second position occurs according to relational movement of the outer and inner tubes, and thus other embodiments could include inner tube 20 being moved in a linear (distal or proximal) direction.

Once blade 30 is locked onto cutting instrument 100 (Figure 4), a cutting or drilling operation, for example, a retrograde drilling step, may be subsequently carried, as known in the art. According to an exemplary embodiment only, the cutter of the present invention may be employed in a retrograde manner to form a recipient socket (at the location of an osteochondral lesion developed on the head of the tibia, for example, or to accommodate retrograde fixation of a graft within two sockets). For example, and as detailed below, Figure 10 illustrates cutter 100 of Figures 1-4 in the vicinity of femur 91 and tibia 93 of knee 99.

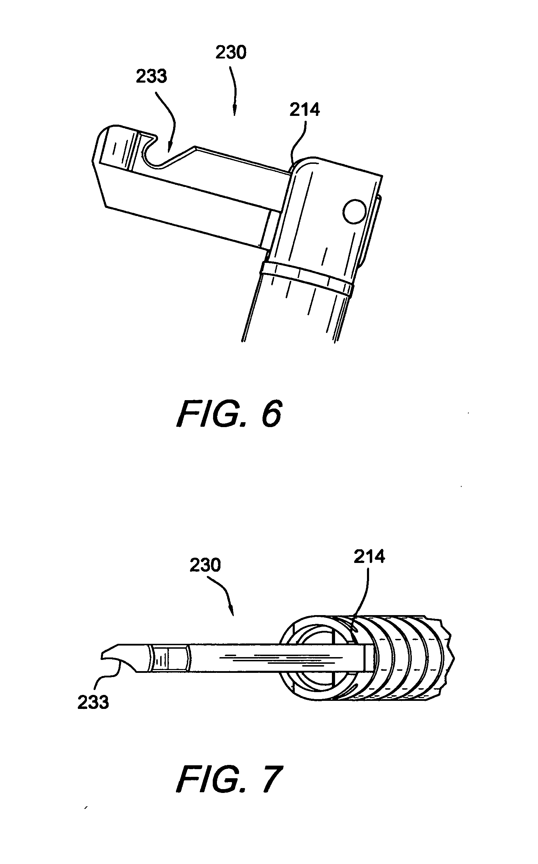

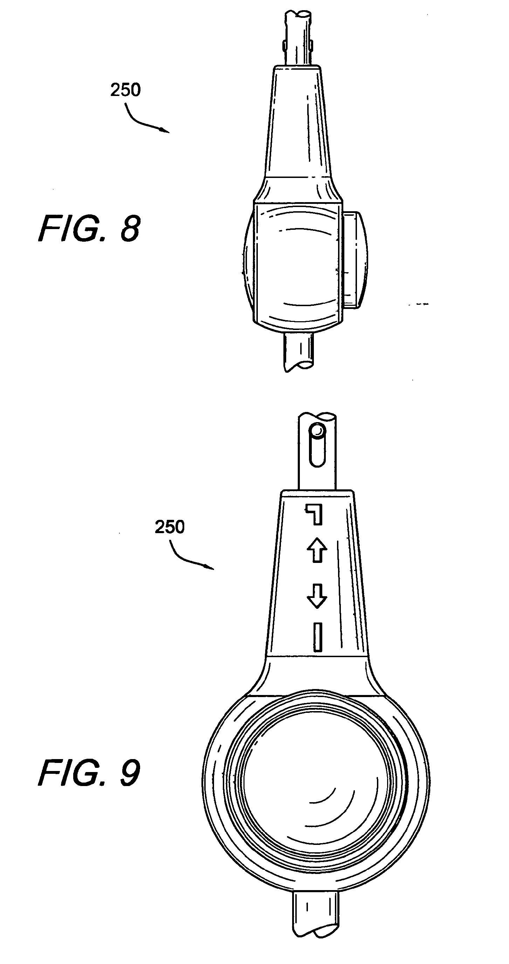

Figures 5-9 illustrate flip cutter 200 according to another embodiment of the: present invention. Flip cutter 200 is similar to the flip cutter 100 of Figures 1-4, but differs in that the handle of cutter 200 includes a push button mechanism 250 (illustrated in detail in Figures 8 and 9) for deployment of blade 230. Flip cutter 200 also differs from the Flip cutter 100 of Figures 1-4 in that blade 230 is provided with a suture passing notch 233 (illustrated in morse detail in Figures 6: and 7). As in the previously-descrihed embodiment; outer tube 210 of the cutter 200 is provided at its most distal end with a cutout 214 that allows blade 230 to pivot from an angle of about zero degrees relative to a longitudinal axis of the tube 210 to arn angle of about ninety degrees relative to the longitudinal axis of the tube 210.

Push button mechanism 250 may be configured to advance an inner or outer tube of a body 210 of Hip cutter 200 (i.e., similar to shaft 20 and tube 10 of flip cutter 100 in Figures 1-4) in a linear direction (for example, a distal direction) to rotate and lock blade-230 in a "flip" position using a mechanism similar to the configuration described above with regard to flip cutter 100. In another exemplary embodiment, push button mechanism 250 may lock cutter 200 and present relational movement of the inner and outer tubes until push button mechanism 250 is engaged.

An exemplary method of ACL reconstruction may be performed according to an embodiment of the present invention by employing cutter 100 (Figures 1-4) or cutter 200 (Figures 5-9 ) to form at least one of a femoral and tibial socket. For example, with cutter 100 oriented as shown in Figure 10, a tibial socket or tunnel is formed within tibia 93 in a retrograde manner.

The present invention may be used to form various sockets or tunnels to allow fixation of a graft (for example, a semitendonosus allograft) or to allow replacement of osteochondral cores or implants in a retrograde manner, to obviate inserting harvesters into the joint. For example, cutting instrument 100, 200 of the present invention may be employed for the formation of sockets duping an "All-Inside ACL RetroConstruction" for ligament repair, developed by Arthrex, Inc. of Naples, Florida (and disclosed in U.S. Patent Application PubJ. No. 2009/0275950), which comprises, for example, the steps of: (i) drilling at least one of a femoral and tibial tunnel or socket using the cutting instrument 100, 200 of Figures, 1-9; (ii) providing a graft (soft tissue graft or BTB graft) in the vicinity of the sockets,; and (iii) securing the graft within the femoral and tibial tunnels (sockets).

According to yet another embodiment, an exemplary method of ACL retrograde reconstruction of the present invention comprises, for example, the steps of: (i) drilling a femoral socket; (ii) drilling a tibial tunnel or socket using a retrodrill technique employing the cutting instrument 100,200 of Figures 1-9; (iii) providing a graft (soft tissue graft or BTB graft) in the vicinity of the sockets; (iv) securing the graft (soft tissue graft or BTB graft) to a continuous loop/button construct comprising a button with an oblong configuration and provided with an inside eyelet that allows the passage of the continuous loop, preferably a suture loop; (v) passing the graft with the button through the femoral tunnel; (vi) securing the button to the femoral cortex once the button exits the femoral socket; and (vii) securing the graft in the tibial tunnel or socket.

Although the present invention has been described in connection with preferred embodiments, many modifications and variations will become apparent to those skilled in the art. While preferred embodiments of the invention have been described and illustrated above, it should be understood that these are exemplary of the invention and are not to be considered as limiting.

## Claims

1. A retrograde cutter comprising:
an elongated body having a distal end (12), a proximal end, and a longitudinal axis, said body further comprising an outer tube (10, 210) and an inner tube (20) housed by said outer tube;
a blade (30, 230) at said distal end of said body, wherein said blade (30, 230) is configured to rotate from a first position generally aligned with said longitudinal axis to a second, flip position which is not aligned with said longitudinal axis; and
a mechanism (15) connecting said blade to distal ends of both said outer and inner tubes, wherein said mechanism comprises a pin and a slot that allow conversion of linear movement of the inner tube (20) into rotational movement of the blade (30, 230) is configured to rotate said blade to said second, flip position upon linear movement of one of said the inner tube (20) and outer tubes in relation to the other of said inner and outer tubes (10, 210),
**characterised in that**
in the second, flip position, the blade (30, 230) is articulated to an angle of about 90 degrees to the longitudinal axis of the elongated body and faces the proximal end of the elongated body for retrograde drilling of a bone tunnel or socket and wherein the blade is locked by the mechanism in the second, flip position.

2. The retrograde cutter of claim 1, said mechanism further comprising:
a first pin (16b) connecting said blade (30) to said outer tube (10); and
a second pin (17b) connecting said blade (30) to said inner tube (20).

3. The retrograde cutter of claim 2, said blade further comprising a second pin hole (17a) for connecting said blade to said second pin (17b), said second pin hole (17a) permitting rotational and sliding movement of said blade (30) relative to said second pin (17b).

4. The retrograde cutter of claim 3, said blade (30) further comprising a first pin hole (16a) for connecting said blade to said first pin (16b), said first pin hole permitting rotational movement of said blade relative to said first pin.

5. The retrograde cutter of claim 1, wherein said mechanism (15) is configured to rotate said blade (30, 230) to said second position upon linear movement of said outer tube (10, 210) in a distal direction in relation to said inner tube (20).

6. The retrograde cutter of claim 5, said inner and outer tubes further comprising threads for locking said inner and outer tubes to prevent relational linear movement.

7. The retrograde cutter of claim 1, further comprising a button mechanism (250) at said proximal end of said body, wherein said button is configured to control said relational linear movement of said inner or outer tube.

8. The retrograde cutter of claim 7, wherein said button is configured to prevent relational movement of the inner and outer tubes until said button mechanism (250) is engaged.

9. The retrograde cutter of claim 7, further comprising a handle on said proximal end of said body, wherein said button is on said handle.

10. The retrograde cutter of claim 1, said blade (230) further comprising a notch (233) for holding a suture material.

## Patentansprüche

1. Rückläufige Schneidevorrichtung, umfassend:
einen langgestreckten Körper, der ein distales Ende (12), ein proximales Ende und eine Längsachse aufweist, wobei der Körper ferner eine äußere Röhre (10, 210) und eine in der äußeren Röhre aufgenommene innere Röhre (20) aufweist;
eine Klinge (30, 230) an dem distalen Ende des Körpers, wobei die Klinge (30, 230) dazu ausgebildet ist, aus einer ersten Position, im Wesentlichen fluchtend mit der Längsachse, in eine zweite, gedrehte Position zu drehen, welche nicht mit der Längsachse fluchtet;
einen Mechanismus (15), der die Klinge mit den distalen Enden sowohl der äußeren als auch der inneren Röhre verbindet, wobei der Mechanismus einen Stift und eine Nut aufweist, die eine Umsetzung einer linearen Bewegung der inneren Röhre (20) in eine Drehbewegung der Klinge (30, 230) erlauben, und
dazu ausgebildet ist, die Klinge in die zweite, gedrehte Position bei einer linearen Bewegung der inneren Röhre (20) oder der äußeren Röhre in Bezug auf die andere der inneren oder äußeren Röhre (10, 210) zu drehen,
**dadurch gekennzeichnet, dass**
in der zweiten, gedrehten Position die Klinge (30, 230) unter einem Winkel von etwa 90 Grad zu der Längsachse des langgestreckten Körpers gelenkig gelagert ist und dem proximalen Ende des langgestreckten Körpers zum rückläufigen Bohren eines Knochentunnels oder einer Höhle gegenüberliegt, und wobei die Klinge durch den Mechanismus in der zweiten, gedrehten Position verriegelt ist.

2. Rückläufige Schneidevorrichtung nach Anspruch 1, ferner umfassend:
einen ersten Stift (16b), der die Klinge (30) mit der äußeren Röhre (10) verbindet; und
einen zweiten Stift (17b), der die Klinge (30) mit der inneren Röhre (20) verbindet.

3. Rückläufige Schneidevorrichtung nach Anspruch 2, wobei die Klinge ferner ein zweites Stiftloch (17a) zum Verbinden der Klinge mit dem zweiten Stift (17b) aufweist, wobei das zweite Stiftloch (17a) eine Dreh- und Gleitbewegung der Klinge (30) bezüglich des zweiten Stifts (17b) zulässt.

4. Retrograde Schneidevorrichtung nach Anspruch 3, wobei die Klinge (30) ferner ein erstes Stiftloch (16) zum Verbinden der Klinge mit dem ersten Stift (16b) aufweist, wobei das erste Stiftloch eine Drehbewegung der Klinge bezüglich des ersten Stifts zulässt.

5. Rückläufige Schneidevorrichtung nach Anspruch 1, wobei der Mechanismus (15) dazu ausgebildet ist, die Klinge (30, 230) in die zweite Position aufgrund einer linearen Bewegung der äußeren Röhre (10, 210) in eine distale Richtung bezüglich der inneren Röhre (20) zu drehen.

6. Rückläufige Schneidevorrichtung nach Anspruch 5, die innere und äußere Röhre weiter Gewinde zum Verriegeln der inneren und äußeren Röhre aufweisen, um eine lineare Bewegung zueinander zu verhindern.

7. Rückläufige Schneidevorrichtung nach Anspruch 1, ferner umfassend einen Druckkopfmechanismus (250) an dem proximalen Ende des Körpers, wobei der Druckknopf dazu ausgebildet ist, die lineare Bewegung der inneren oder äußeren Röhre zueinander zu steuern.

8. Rückläufige Schneidevorrichtung nach Anspruch 7, wobei der Druckknopf dazu ausgebildet ist, eine Bewegung der inneren und äußeren Röhre zueinander zu verhindern, bis der Druckknopfmechanismus (250) eingerückt ist.

9. Rückläufige Schneidevorrichtung nach Anspruch 7, weiter umfassend einen Griff an dem proximalen Ende des Körpers, wobei der Druckknopf an dem Griff angebracht ist.

10. Rückläufige Schneidevorrichtung nach Anspruch 1, wobei die Klinge (230) weiter eine Einkerbung (233) zum Halten eines Fadenmaterials aufweist.

## Revendications

1. Dispositif de découpe rétrograde comprenant :
✔ un corps allongé ayant une extrémité distale (12), une extrémité proximale et un axe longitudinal, ledit corps comprenant en outre un tube externe (10, 210) et un tube interne (20) logé par ledit tube externe ;
✔ une lame (30, 230) au niveau de ladite extrémité distale dudit corps, où ladite lame (30, 230) est configurée pour tourner d'une première position généralement alignée avec ledit axe longitudinal à une seconde position, de basculement, qui n'est pas alignée avec ledit axe longitudinal ; et
✔ un mécanisme (15) reliant ladite lame aux extrémités distales desdits tubes externe et interne, où ledit mécanisme comprend une broche et une fente qui permettent la conversion du mouvement linéaire du tube interne (20) en un mouvement de rotation de la lame (30, 230), est configuré pour faire tourner ladite lame dans ladite seconde position, de basculement, lors d'un mouvement linéaire de l'un desdits tubes interne (20) et tubes externes par rapport à l'autre desdits tube interne et externe (10, 210), **caractérisé en ce que** dans la seconde position de basculement, la lame (30, 230) est articulée à un angle d'environ 90 degrés par rapport à l'axe longitudinal du corps allongé et est en regard de l'extrémité proximale du corps allongé pour un forage rétrograde d'un tunnel osseux ou d'une cavité osseuse et où la lame est verrouillée par le mécanisme dans la seconde position de basculement.

2. Dispositif de découpe rétrograde selon la revendication 1, ledit mécanisme comprenant en outre :
✔une première broche (16b) reliant ladite lame (30) audit tube externe (10) ; et
✔ une seconde broche (17b) reliant ladite lame (30) audit tube interne (20).

3. Dispositif de découpe rétrograde selon la revendication 2, dans lequel ladite lame comprend en outre un trou de seconde broche (17a) permettant de relier ladite lame à ladite seconde broche (17b), ledit trou de seconde broche (17a) permettant un mouvement de rotation et de glissement de ladite lame (30) par rapport à ladite seconde broche (17b).

4. Dispositif de découpe rétrograde selon la revendication 3, dans lequel ladite lame (30) comprend en outre un trou de première broche (16a) permettant de raccorder ladite lame à ladite première broche (16b), ledit trou de première broche permettant un mouvement de rotation de ladite lame par rapport à ladite première broche.

5. Dispositif de découpe rétrograde selon la revendication 1, dans lequel ledit mécanisme (15) est configuré pour faire tourner ladite lame (30, 230) dans ladite seconde position lors d'un mouvement linéaire dudit tube externe (10, 210) dans une direction distale par rapport audit tube interne (20).

6. Dispositif de découpe rétrograde selon la revendication 5, dans lequel lesdits tubes interne et externe comprennent en outre des filets pour le verrouillage desdits tubes interne et externe en vue d'empêcher un mouvement linéaire relationnel.

7. Dispositif de découpe rétrograde selon la revendication 1, comprenant en outre un mécanisme à bouton (250) au niveau de ladite extrémité proximale dudit corps, où ledit bouton est configuré pour commander ledit mouvement linéaire relationnel dudit tube interne ou externe.

8. Dispositif de découpe rétrograde selon la revendication 7, dans lequel ledit bouton est configuré pour empêcher un mouvement relationnel des tubes interne et externe jusqu'à ce que ledit mécanisme à bouton (250) soit enclenché.

9. Dispositif de découpe rétrograde selon la revendication 7, comprenant en outre un manche sur ladite extrémité proximale dudit corps, où ledit bouton se trouve sur ledit manche.

10. Dispositif de découpe rétrograde selon la revendication 1, dans lequel ladite lame (230) comprend en outre une encoche (233) permettant de maintenir un matériau de suture.
